**Europäisches Patentamt**

**European Patent Office**

**Office européen des brevets**

(19)

(11) Publication number: **0 137 725**
**B1**

(12) # EUROPEAN PATENT SPECIFICATION

(45) Date of publication of patent specification: **18.05.88**

(21) Application number: **84306125.0**

(22) Date of filing: **07.09.84**

(51) Int. Cl.⁴: **A 61 F 13/18**

(54) **Absorbent body with fluid transport means.**

(30) Priority: **08.09.83 US 530320**

(43) Date of publication of application:
**17.04.85 Bulletin 85/16**

(45) Publication of the grant of the patent:
**18.05.88 Bulletin 88/20**

(84) Designated Contracting States:
**AT BE DE FR GB IT LU NL SE**

(56) References cited:
**DE-A-2 656 482**
**GB-A-2 082 643**
**US-A-3 017 304**
**US-A-3 938 522**

(73) Proprietor: **PERSONAL PRODUCTS COMPANY**
**Van Liew Avenue**
**Milltown New Jersey 08850 (US)**

(72) Inventor: **Chapas, Richard B.**
**Two Stanford Ct.**
**East Windsor New Jersey 98520 (US)**
Inventor: **Mavinkurve, Pramod**
**Nine Dov Place**
**Kendall Park New Jersey 08824 (US)**

(74) Representative: **Jones, Alan John et al**
**CARPMAELS & RANSFORD 43 Bloomsbury**
**Square**
**London, WC1A 2RA (GB)**

Courier Press, Leamington Spa, England.

# 0 137 725

**Description**

This invention relates to providing a pad of fibrous absorbent material for absorbing body fluids and in particular relates to an absorbent pad for use in such products as sanitary napkins, panty liners and diapers.

In general, such products comprise one or more layers of a core of hydrophilic material such as wood pulp, rayon or tissue. The hydrophilic material, generally fibrous in form, is provided as a pad having a rectangular or oblong shape or, in some cases, a shape designed to more closely fit the anatomy of the wearer. The pad is usually provided with an enveloping cover pervious to body fluids on the side of the pad designed to be placed against the body and impervious to such fluids on the side facing away from the body. The object of such body fluid impervious cover is, of course, to protect the clothing from staining and wetting.

In the main, such products have satisfactorily performed their function of absorbing and retaining body fluids and preventing staining and wetting of the wearer's clothing. Generally, when the product is properly placed and retained by the wearer in its intended position, body fluid is directed at or near the center of the product and distributes, by means of liquid wicking, throughout the absorbent medium. Unfortunately, in a significant number of cases, the product is misplaced, either initially by the user or because of displacement caused by the activities of the user. In these circumstances, body fluid will strike the pad off-center and closer to the peripheral edges of the pad. It is believed that this off-center disposition of body fluid is the cause of a significant number of failures associated with the use of these products, i.e. body fluid staining and wetting the clothing of the wearer.

Accordingly, there is a need for providing a pad for body fluid absorbent products which protects the user from the disadvantageous effects of product misplacement.

GB—A—2 082 643 discloses an absorbent body of fibrous material for absorbing body fluids, said absorbent body being generally planar and elongated and having a relatively dense peripheral edge portion integral with the remainder of said absorbent body.

In accordance with the present invention, an absorbent pad for a body fluid absorbent product is provided which alleviates the problem of wetting or staining the clothing of the wearer by body fluid which strikes the pad in proximity to the peripheral edges.

The pad of the present invention is characterised in that said relatively dense peripheral edge portion is at least the longitudinal center portion of the absorbent body, whereby body fluid striking the absorbent body at a point in proximity to the relatively dense peripheral edge will be transported rapidly away from said point.

This feature is preferably accomplished by compressing the appropriate portion of the peripheral edge. Preferably such compressed edge is densified to a bulk density of at least ten times that of the least dense portion of the pad. More preferably, the relatively dense peripheral edge portion of the pad has a bulk density at least twenty times that of the least dense portion of the pad.

In a preferred embodiment, the pad is provided with a density gradient wherein the density gradually increases from the least dense portion of the pad toward the relatively dense peripheral edge portion of the pad. The gradual increase however becomes a sharp increase as the periphery is approached and ultimately the densities at the peripheral edge are those prescribed above. It is preferred that the compressed peripheral edge entirely circumscribe the pad, although a substantial realization of the benefits of this invention are accrued when only a portion of the peripheral edges are of the herein prescribed densities. In a specific embodiment all or a portion of the edges of the pad are overlaid with a fluid impervious cover to shield the user's clothing from wetting.

It has been discovered that by following the teachings of this invention, fluid reaching a point at the peripheral edge of the pad is rapidly transported along the periphery and away from such point. This transport of fluid has been experimentally observed in *in-vitro* tests in which products incorporating such pad show far fewer incidence of failing, i.e. body fluid appearing on the outer surface of such products.

In the accompanying drawings:

Figure 1 is a perspective view of a sanitary napkin as a first embodiment of this invention with parts removed therefrom to show internal detail;

Figure 2 is a transverse, cross-sectional view of the sanitary napkin of Figure 1 taken along line 2—2 of Figure 1;

Figure 2a is a graphical representation of the pad density gradient in the transverse direction of the pad;

Figure 3 is a longitudinal, cross-sectional view of the sanitary napkin of Figure 1, taken along line 3—3 of Figure 1; and

Figure 3a is a graphical representation of the pad density gradient in the longitudinal direction of the pad.

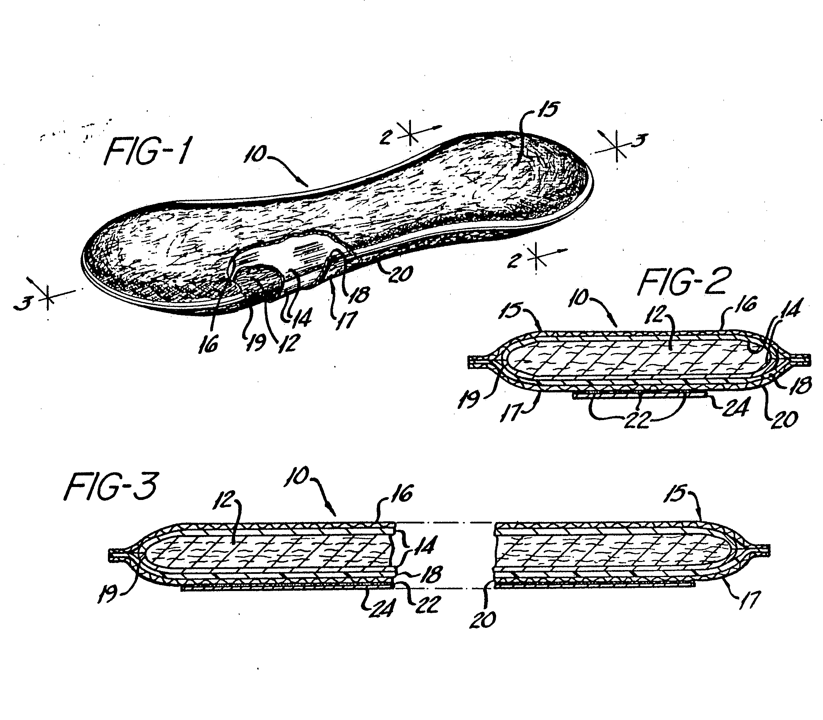

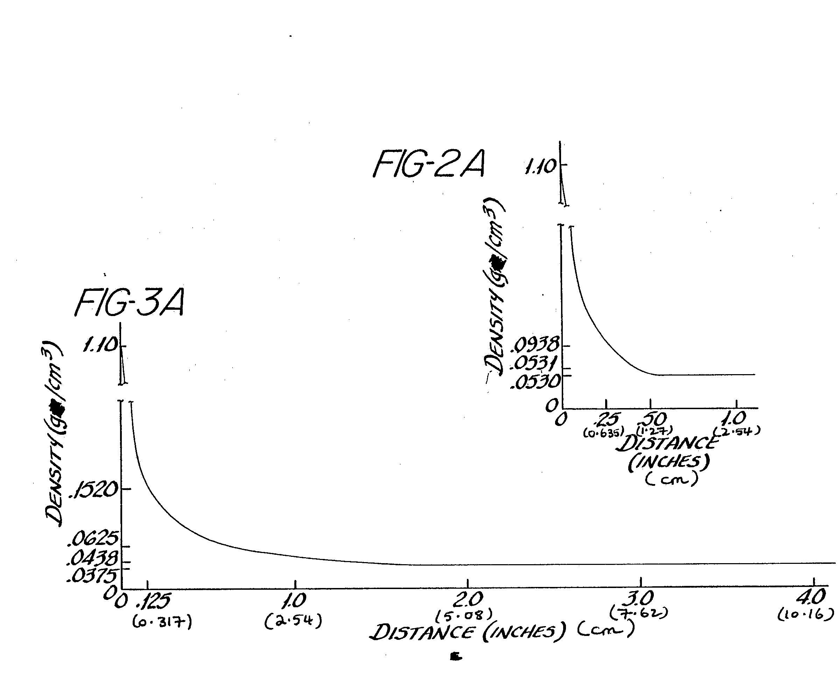

Referring now to Figure 1, illustrated there, in perspective view, is a sanitary napkin 10 according to this invention. Figures 2 and 3 illustrate the napkin 10 in transverse and longitudinal cross sections, respectively.

The napkin consists of an absorbent element 12 which is shown in the form of a generally planar pad. The choice of materials for use in the pad of this invention is not critical. It is important, however, that the

2

# 0 137 725

pad employed be capable of densification in the manner prescribed herein and that such densification result in the fluid transport properties described in greater detail hereinafter.

Preferably, the pad comprises loosely associated absorbent hydrophilic material such as cellulose fibers, e.g. wood pulp, regenerated cellulose or cotton fibers. Such fibers may be chemically or physically modified and the pad may include such fibers in combination with other materials, both natural and synthetic, such as hydrophilic foams or hydrophilic polymers. A material of choice is wood pulp which is inexpensive, readily available and lends itself well to the densification specified herein.

The pad 12, as illustrated in Figures 1—3, is wrapped in a tissue wrap 14 which is provided to aid in holding the product together during manufacturing and to help retain the shape of the finished product.

Overlying a first major surface 15 of the napkin 10, i.e. the side of the napkin to be worn against the body of the user, is a body fluid pervious cover 16. The cover 16 may be any woven or nonwoven material pervious to body fluid striking its surface, such covers being well known in the art. In a preferred embodiment, the cover comprises thermoplastic material capable of being fusibly sealed to another element of the napkin, e.g. by heat, pressure or sonic sealing.

A material of choice for the cover is a fabric comprising heat bondable polyester/polyethylene conjugate fibers. Such conjugate fibers are fibers which comprise a polyester core surrounded by a sheath of polyethylene. Preferably, the conjugate fibers employ high density polyethylene, that is linear polyethylene that has a density of at least 0.94 g/cc and a Melt Index (as determined by ASTMD-1288E method, employing the parameters of 190°C and 2160 g) of greater than 1, preferably greater than 10, and more preferably from 20 to 50. The fibers may comprise from 40 to 60 percent, by weight polyester and, preferably, from 45 to 55 percent by weight polyester, with the remainder being polyethylene. Such fibers may be used in deniers of from 1 to 6 (0.11 to 0.66 Tex) and may be from 1/2 inch (1.27 cm) to 3 to 4 inches (7.62 to 10.16 cm) long. The fabric comprising such fibers is stabilized by applying heat thereto under essentially zero pressure whereby thermal bonding takes place without destroying the integrity of the fibers.

Overlying the second major surface 17 of the pad 12 (the side worn away from the body of the user) and at least a part of the edges 19 of pad 12 is a body fluid impervious layer 18. The layer 18 is provided to preclude body fluid from passing onto an undergarment and may be constructed of any material suitable for this purpose. For example, the layer 18 may be a polymeric film such as polyethylene, polypropylene, or cellophane or may be a normally fluid pervious material that has been treated to be impervious, such as a fluid repellent paper.

Advantageously, the layer 18 is a heat bondable material, such as polyethylene, which can be bonded to layer 16 to completely enclose pad 12.

In a preferred configuration, layer 20, which comprises a nonwoven fabric, constitutes the outer layer of the garment side of the napkin 10. This fabric outer layer is provided for aesthetic purposes and for its soft feel.

As best viewed in Figures 2 and 3, the garment surface of the napkin 10 is provided with pressure sensitive adhesive elements 22 for adhering the napkin to the crotch portion of the wearer's undergarment. As shown in this specific embodiment these adhesive elements 22 are in the form of three longitudinally extending bands, although it will be understood by those skilled in the art that many variations in the number and shape of these adhesive elements are possible. The pressure-sensitive adhesive may be any of the already known compositions suitable for this purpose including the water based pressure-sensitive adhesives, such as the acrylate adhesives, e.g. vinyl acetate-2 ethyl hexyl acrylate copolymer, which are generally combined with tackifiers such as ethylene amine. Alternatively, the adhesive may comprise the rapid setting thermoplastic (hot melt) adhesives such as block copolymers exemplified by styrene and butadiene/styrene copolymers. The adhesive elements may also comprise a two-sided adhesive tape.

The adhesive areas are protected by a release strip 24 to avoid undesired adhesion prior to use. The release strip 24 may be made of any suitable sheet-like material which adheres with sufficient tenacity to the adhesive elements 22 to remain in place, but which can be readily removed when the napkin 10 is to be used. A particularly useful material is a semi-bleached kraft paper, the adhesive contacting side of which has been silicone coated to provide for easy removal from the adhesive just prior to use.

Referring now to Figure 2a, shown graphically is a plot of density as the ordinate versus the transverse distance along the line 2—2 taken through pad 12 illustrated in Figure 2, as the abscissa. As can be seen, the density of the pad increases gradually and at a slow rate from a point in the pad of least density toward the outer periphery of the pad. In the embodiment illustrated, the least dense portion of the pad, with respect to the transverse and longitudinal centerlines, is the center of the pad. It should be understood however that this embodiment is merely exemplary and that, for example, a compressed central channel may be provided in the pad, in which case the least dense portion of the pad would be off-center.

In accordance with the teachings of this invention, and as best illustrated in Figure 2a, as the periphery of the pad is closely approached, the density of the pad increases sharply so that at the extreme peripheral edge, the density is at least ten times that of the least dense portion and preferably, at least twenty times. For the embodiment illustrated in the drawings the density of the peripheral edge is 1.0 g/cc, and is 20 times that of the least dense portion of the pad, which density is 0.05 g/cc. It will be understood by those skilled in the art that the density being referred to herein is the local bulk density of the pad, i.e. the weight of a unit

3

volume of pad, including in such unit volume the interstitial void volume such as exists between wood pulp fibers, for example.

Referring now to Figure 3a, illustrated there is a plot of density as the ordinate versus longitudinal distance along the line 3—3, taken through pad 12 illustrated in Figure 3 as the abscissa. Once again it can be seen that the density increases gradually at a slow rate from the least dense portion of the pad (in this embodiment, the center) toward the periphery. As the periphery is closely approached the density increases sharply to the values prescribed herein.

In a preferred embodiment, the high density peripheral edge area extends inwardly from the extreme periphery a distance of at least 0.1 cm and preferably at least 0.2 cm. Within the peripheral edge area, the densities are within the prescribed values of at least ten times that of the least dense area of the pad and preferably at least twenty times that of the least dense areas. While substantial benefit is gained by having only the longitudinally central peripheral edge portion of the pad provided with the prescribed high density, it is preferable that the entire peripheral areas of the pad be so densified.

The advantages of this invention will be best understood by considering the following examples.

Comparative Example

A pad is provided comprising 8.5 g of southern pine semi-bleached wood pulp and having the dimensions of 19.6 cm long, 6.6 cm wide, and 1.65 cm thick. The pad, representative of prior art pads utilized in sanitary napkins now on the market, has an essentially uniform density of 0.50 g/cc. The pad is covered with a tissue wrap on its side.

To simulate the disadvantageous effect of off-center liquid depositions, liquid is deposited on the body side of the pad at a point 1.27 cm inward from a longitudinal edge of the pad and on the transverse center line. The liquid deposited is an ersatz menstrual fluid which comprises 1% by weight aqueous NaCl solution and 0.2%, by weight, of surface active agent to emulate the surface tension of menstrual fluid, i.e. approximately 50 dynes/cm ($5 \times 10^{-2}$ N/m). The surface active agent used is poly(ethylene oxide)-poly-(propylene oxide) block copolymer sold by the Wyandette Chemical Corporation of Michigan, U.S.A. under the tradename Pluronic®. The liquid also comprises a red dye. The liquid is deposited at the above described point of liquid deposition by placing the tip of a 50 cubic centimeter burette as close as possible to the pad without actually touching the pad. Ten cubic centimeters of the fluid are then deposited as quickly as can be discharged from the burette (utilizing a full burette with the stop cock in the completely open position).

It is observed that the liquid tends to remain at the point of deposition with only very slow migration to adjacent parts of the pad. After a substantial portion of the ten cubic centimeters of liquid has been deposited, the liquid tends to puddle and run off the surface of the pad. Such a condition, in a complete napkin, would cause a napkin failure, i.e. menstrual fluid wetting the undergarment of the user. This is true even when the longitudinal edges of the pad are covered by a fluid impervious cover such as is illustrated in Figures 1—3 in that the puddled fluid will run over the edge of the impervious layer and wet the user's undergarment.

Example 1

A series of pads are provided having the general configuration of pad 12 illustrated in Figures 1—3. The pads, comprising southern pine semi-bleached wood pulp, measure 19.2 cm long and 5.1 cm wide at the longitudinal center, and have the density gradient set out in Figures 2a and 3a.

The pads are subjected to the same deposition of ersatz menstrual fluid as is described in connected with the comparative example. The pads are completely enveloped with a tissue wrap as is illustrated in Figures 1—3. One pad is identical to that shown in Figures 1 to 3, whereas another pad is provided with a compressed central channel. Accordingly, the least compressed parts of this pad along a transverse, longitudinally centered line is at a point between the compressed central channel and the compressed peripheral area. Unlike the prior art pads, there is a rapid and advantageous transport of liquid away from the point of deposition. By observation it is noted that the transport is rapid enough to prevent liquid puddling at the point of deposition.

Example 2

A series of pads are provided, identical to those of Example 1 with the exception that only the body sides of the pads are covered with tissue. Again it was noted that rapid transport of liquid results.

Example 3

A series of pads are provided identical to those of Example 1 with the exception that no tissue cover is employed. Again it was noted that rapid transport of liquid results.

Example 4

To quantify the results exhibited by the pads of Examples 1 to 3, the deposition of fluid was carried out, as described in Examples 1—3 and in the comparative example, for a number of pads having the various described configurations. The initial spread of liquid along the peripheral edge of the pad was measured immediately after deposition of liquid and then five minutes thereafter. The arithmetic average of these values for each type of pad are reported in Table 1 below.

TABLE 1

| Pad type | No. of pads | Initial length in. (cm) | Length after 5 min in. (cm) |
|---|---|---|---|
| Comparative example | 9 | 1.44 (3.66) | 2.35 ( 5.97) |
| Example 1—Tissue on Both sides | 12 | 2.75 (6.98) | 6.27 (15.93) |
| Example 2—Tissue on Body side Only | 15 | 2.76 (7.01) | 6.60 (16.76) |
| Example 3—No tissue | 15 | 2.28 (5.79) | 7.05 (17.91) |

As can be seen from Table 1, the pads of Examples 1—3 according to this invention all exhibit a greater initial length, i.e. more rapid liquid transport, than the prior art pad of the comparative example. After five minutes the contrast is even greater.

Example 5

A series of pads of Example 1 are incorporated into a sanitary napkin having the general structure shown in Figures 1—3. The napkins are then tested on a Dynamic Form Testing apparatus, the test comprising suspending the napkin to be tested across a rubber mold which simulates the female form. The form is set in motion by means of a set of gears, cams and rods and ersatz menstrual fluid is allowed to drip onto the napkin to closely approximate in-use conditions. The fluid is applied at a rate of 0.2 cc/min and the form is operated at a speed of 60 cycles/min. The fluid capacity of the napkin under dynamic conditions is measured by the total volume of fluid applied at the time of failure, i.e. the time at which spotting is noted on the underside of the napkin.

A second series of napkins incorporating the prior art pad described in the comparative example are similarly tested. The results of these tests are set out in Table 2 below.

TABLE 2

| Pad type | No. of pads | Pad weight (g) | Total napkin wt (g) | Dynamic capacity (cc) |
|---|---|---|---|---|
| Comparative example | 6 | 8.53 | 12.9 | 50.9 |
| Example 1 | 6 | 8.62 | 11.0 | 84.4 |

As can be seen from Table 2, the capacity of the napkin as measured by this test is substantially increased.

The absorbent bodies of this invention may be manufactured by several alternative methods which will occur to one skilled in the art. For example, a pad may be provided which is thicker at the peripheral portions than at the central portions and then compressed to a uniform thickness to produce the prescribed density gradient. Preferably, a pad is first provided of uniform thickness and is compressed in a mold to produce a compressed pad wherein the peripheral portions are thinner and denser than the central portions to result in the prescribed gradient. The pressure applied to the pad may be uniform or excessive pressure may be brought to bear against the extreme peripheral edges. Such excessive pressure may be applied, for example, by the use of die cutters which cut the pad to the desired shape while simultaneously applying the excessive pressure which, in cooperation with the contours of the mold, produce the herein prescribed density gradient.

**Claims**

1. An absorbent body of fibrous material (12) for absorbing body fluids, said absorbent body (10) being generally planar and elongated and having a relatively dense peripheral edge portion integral with the remainder of said absorbent body (10), characterised in that said relatively dense peripheral edge portion is at least the longitudinal center portion of the peripheral edge of the body, whereby body fluid striking the absorbent body (10) at a point in proximity to the relatively dense peripheral edge will be transported rapidly away from said point.

2. The absorbent body of claim 1 wherein the density of said relatively dense peripheral edge portion is at least ten times greater than the least dense portions of said absorbent body (10).

3. The absorbent body of claim 2 wherein the density of said relatively dense peripheral edge portion is at least twenty times greater than the least dense portion of said absorbent body (10).

4. The absorbent body of claim 2 or claim 3 wherein said relatively dense peripheral edge portion extends inwardly from the extreme periphery of said absorbent body (10) for a distance of at least 0.1 cm.

5. The absorbent body of claim 4 wherein said relatively dense peripheral edge portion extends inwardly from the extreme peripheral of said absorbent body (10) for a distance of at least 0.2 cm.

6. The absorbent body of any one of claims 1 to 5 wherein said relatively dense peripheral edge portion is essentially the entire peripheral edge of said absorbent body (10).

7. The absorbent body of any one of claims 1 to 6 wherein the density of the absorbent body (10) increases from the least dense portion of said absorbent body (10) to the relatively dense peripheral edge portion.

8. The absorbent body of claim 7 wherein the rate of increase in density with respect to distance from the least dense portion of said absorbent body to the relatively dense peripheral edge portion is low and then increases as the relatively dense peripheral edge portion approached.

9. The absorbent body of any one of claims 1 to 8 wherein said relatively dense peripheral edge portion is at least partially covered by a fluid impervious cover (18).

10. The absorbent body of any one of claims 1 to 9 incorporated into a sanitary napkin or dispensible diaper.

**Patentansprüche**

1. Absorbierender Körper aus faserigem Material (12) zum Absorbieren von Körperflüssigkeiten, wobei der genannte absorbierende Körper (10) im allgemeinen eben und langgestreckt ist und einen relativ dichten, mit dem Rest des genannten absorbierenden Körpers (10) einstückig verbundenen Umfangsrandteil aufweist, dadurch gekennzeichnet, daß der genannte, relativ dichte Umfangsrandteil wenigstens der sich in der Längsrichtung erstreckende Mittelteil des Umfangsrandes des Körpers ist, wodurch Körperflüssigkeit, die auf den absorbierenden Körper (10) an einem Punkt auftrifft, der sich in unmittelbarer Nähe des relativ dichten Umfangsrandes befindet, von diesem Punkt rasch wegtransportiert werden wird.

2. Absorbierender Körper nach Anspruch 1, worin die Dichte des genannten, relativ dichten Umfangsrandteiles wenigstens das 10-fache der Dichte des am wenigsten dichten Teiles des genannten absorbierenden Körpers (10) beträgt.

3. Absorbierender Körper nach Anspruch 2, worin die Dichte des genannten, relativ dichten Umfangsrandteiles wenigstens das 20-fache der Dichte des am wenigsten dichten Teiles des genannten absorbierenden Körpers (10) beträgt.

4. Absorbierender Körper nach Anspruch 2 oder 3, worin sich der genannte, relativ dichte Umfangsrandteil von dem äußersten Rand des genannten absorbierenden Körpers (10) über einen Abstand von wenigstens 0,1 cm nach innen erstreckt.

5. Absorbierender Körper nach Anspruch 4, worin sich der genannte, relativ dichte Umfangsrandteil von dem äußersten Rand des genannten absorbierenden Körpers (10) über einen Abstand von wenigstens 0,2 cm nach innen erstreckt.

6. Absorbierender Körper nach einem der Ansprüche 1 bis 5, worin der genannte, relativ dichte Umfangsrandteil im wesentlichen der gesamte Umfangsrand des genannten absorbierenden Körpers (10) ist.

7. Absorbierender Körper nach einem der Ansprüche 1 bis 6, worin die Dichte des absorbierenden Körpers (10) von dem am wenigsten dichten Teil des genannten absorbierenden Körpers (10) zu dem relativ dichten Umfangsrandteil hin zunimmt.

8. Absorbierender Körper nach Anspruch 7, worin das Ausmaß, mit dem die Dichte in Bezug auf den Abstand von dem am wenigsten dichten Teil des genannten absorbierenden Körpers zu dem relativ dichten Umfangsrandteil hin zunimmt, gering ist und dann in dem Maße zunimmt, wie der relativ dichte Umfangsrandteil näherrückt.

9. Absorbierender Körper nach einem der Ansprüche 1 bis 8, worin der genannte, relativ dichte Umfangsrandteil wenigstens teilweise durch eine flüssigkeitsundurchlässige Hülle (18) bedeckt ist.

10. Absorbierender Körper nach einem der Ansprüche 1 bis 9, welcher einer Damenbinde oder einer Wegwerfwindel einverleibt ist.

**Revendications**

1. Un corps absorbant en matière fibreuse (12) pour absorber des fluides du corps humain, ledit corps absorbant (10) étant dans l'ensemble plan et allongé et comportant une partie marginale périphérique relativement dense, solidaire du reste dudit corps absorbant (10), caractérisé en ce que ladite partie marginale périphérique relativement dense est au moins la partie centrale longitudinale du bord périphérique du corps, de sorte que du fluide du corps humain entrant en contact avec le corps absorbant (10) en un point situé à proximité du bord périphérique relativement dense soit transféré rapidement en éloignement dudit point.

2. Le corps absorbant selon la revendication 1, dans lequel la densité de ladite partie marginale périphérique relativement dense est au moins dix fois supérieure à celle des parties moins denses dudit corps absorbant (10).

3. Le corps absorbant selon la revendication 2, dans lequel la densité de ladite partie marginale périphérique relativement dense est au moins vingt fois supérieure à celle de la partie la moins dense dudit corps absorbant (10).

4. Le corps absorbant selon la revendication 2 ou la revendication 3, dans lequel ladite partie marginale périphérique relativement dense s'étend vers l'intérieur à partir de la périphérie extrême dudit corps absorbant (10) sur une distance d'au moins 0,1 cm.

5. Le corps absorbant selon la revendication 4, dans lequel ladite partie marginale périphérique relativement dense s'étend vers l'intérieur à partir de la périphérie extrême dudit corps absorbant (10) sur une distance d'au moins 0,2 cm.

6. Le corps absorbant selon l'une quelconque des revendications 1 à 5, dans lequel ladite partie marginale périphérique relativement dense est dans l'essentiel le bord périphérique entier dudit corps absorbant (10).

7. Le corps absorbant selon l'une quelconque des revendications 1 à 6, dans lequel la densité du corps absorbant (10) augmente depuis la partie la moins dense dudit corps absorbant (10) jusqu'à la partie marginale périphérique relativement dense.

8. Le corps absorbant selon la revendication 7, dans lequel le taux d'augmentation de densité en relation avec la distance entre la partie la moins dense dudit corps absorbant et la partie marginale périphérique relativement dense est faible et augmente ensuite à mesure qu'on se rapproche de la partie marginale périphérique relativement dense.

9. Le corps absorbant selon l'une quelconque des revendications 1 à 8, dans lequel ladite partie marginale périphérique relativement dense est recouverte au moins partiellement par un revêtement imperméable aux fluides (18).

10. Le corps absorbant selon l'une quelconque des revendications 1 à 9, incorporé à une serviette hygiénique ou à une couche jetable.

FIG-1

FIG-2

FIG-3

0 137 725

FIG-2A

FIG-3A

0 137 725